(19) Europäisches Patentamt
European Patent Office
Office européen des brevets

(11) **EP 3 005 943 B1**

(12) **EUROPEAN PATENT SPECIFICATION**

(45) Date of publication and mention of the grant of the patent:
**23.09.2020 Bulletin 2020/39**

(21) Application number: **14807402.4**

(22) Date of filing: **17.04.2014**

(51) Int Cl.:
***A61B 5/055*** (2006.01)

(86) International application number:
**PCT/KR2014/003346**

(87) International publication number:
**WO 2014/196732 (11.12.2014 Gazette 2014/50)**

(54) **NOVEL MAGNETIC RESONANCE IMAGE TECHNIQUE FOR IMAGING AND EVALUATING COLLATERAL CIRCULATION**

NEUARTIGE MAGNETRESONANZBILDTECHNIK ZUR BILDGEBUNG UND EVALUIERUNG EINES KOLLATERALKREISLAUFS

NOUVELLE TECHNIQUE D'IMAGE PAR RÉSONANCE MAGNÉTIQUE POUR UNE IMAGERIE ET UNE ÉVALUATION DE CIRCULATION COLLATÉRALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2013 KR 20130063423**

(43) Date of publication of application:
**13.04.2016 Bulletin 2016/15**

(73) Proprietor: **Samsung Life Public Welfare Foundation**
**Seoul 140-893 (KR)**

(72) Inventors:
• **BANG, Oh Young**
  **Seoul 135-505 (KR)**
• **KIM, Suk Jae**
  **Seoul 135-990 (KR)**
• **SON, Jeong Pyo**
  **Uiwang-si**
  **Gyeonggi-do 437-729 (KR)**

(74) Representative: **Kilger, Ute**
**Boehmert & Boehmert**
**Anwaltspartnerschaft mbB**
**Pettenkoferstrasse 22**
**80336 München (DE)**

(56) References cited:
**JP-A- 2005 095 340     JP-A- 2013 085 652**
**JP-B2- 5 171 251     US-A1- 2012 275 676**

• **RONALD WOLF: "CT Imaging and Physiologic Techniques in Interventional Neuroradiology", 1 January 2008 (2008-01-01), INTERVENTIONAL NEURORADIO,, PAGE(S) 87 - 111, XP009192838, * page 101, paragraph "subacute and chronic setting" ***
• **HYANG-I PARK ET AL: "Reduced rCBV Ratio in Perfusion-Weighted MR Images Predicts Poor Outcome after Thrombolysis in Acute Ischemic Stroke", EUROPEAN NEUROLOGY., vol. 65, no. 5, 1 January 2011 (2011-01-01), pages 257-263, XP055329802, Switzerland ISSN: 0014-3022, DOI: 10.1159/000324727**

## Description

## Technical Field

The present invention relates to a novel magnetic resonance imaging technique for imaging and evaluating collateral circulation.

## Background Art

Cerebral infarction is one of the leading causes of death worldwide, and shows a high outcome of disability and the highest death rate, as a single disease, among the causes of death in Korea. A cerebral infarction is a symptom in which the supply of blood to brain tissues is blocked due to cerebrovascular occlusion, resulting in the infarction of the brain tissues. The only way for cerebral infarction therapy is to early recanalize the occluded blood vessel, thereby restoring the cerebral blood flow before the brain tissues reach a complete infarction. The cerebral blood flow is maintained by the collateral circulation supplied from surrounding blood vessels until the occluded brain blood vessel is recanalized. Therefore, the collateral status before recanalization therapy is an important determinant of tissue fate with respect to cerebral infarction [Bang OY et al., J Neurol Neurosurg Psychiatry, 2008; 79:625]. In addition, collateral circulation has a profound effect on the recanalization rates of the occluded blood vessel and hemorrhagic change of brain tissues [Bang OY et al., Stroke, 2011; 42:693; Bang OY et al., Stroke, 2011; 42: 2235]. In other words, it is very important to assess the collateral status at the early stage in making therapeutic decisions and predicting the outcomes in patients with cerebral infarction. Since the cerebral infarction has various causes and therapeutic responses, it is very important to develop image biomarkers using various magnetic resonance imaging techniques. Representative research techniques of cerebral infarction using magnetic resonance imaging are diffusion and perfusion-weighted imaging, fluid attenuation inversion recovery (FLAIR), and the like, and these images and maps made through image post-processing are actively used as image biomarkers in clinical trials. Although these various techniques have been developed, the method of directly imaging collateral circulation using magnetic resonance imaging and evaluating the collateral status has not yet developed.

Generally, digital subtraction angiography (DSA) is currently used as the gold standard for the imaging of collateral circulation and evaluation of collateral status in clinical diagnosis. However, DSA technique is invasive, takes a relatively long time for the implementation, requires expertise, and has a risk of thrombotic complications. Moreover, when the collateral status is evaluated using DSA, the evaluation is not sufficiently implemented until the late venous phase or the evaluation on the contralesional blood vessel is omitted, and thus the collateral evaluation may be restricted. Therefore, novel techniques need to be developed that are non-invasive to the human body and can grasp the collateral status easily and objectively for a shorter time.

Throughout the entire specification, many papers and patent documents are referenced and their citations are represented. The disclosure of cited papers and patent documents, and the level of the technical field within which the present invention falls and details of the present invention are explained more clearly.

## Detailed Description of the Invention

## Technical Problem

The present inventors have endeavored to solve the above-mentioned problems, and as a result, the present inventors have developed a novel method for visualizing collateral circulation and evaluating the collateral status using magnetic resonance imaging, and have completed the present invention.

Accordingly, an aspect of the present invention is to provide a method for imaging collateral circulation in the body.

Other purposes and advantages of the present disclosure will become more obvious with the following detailed description of the invention, claims, and drawings.

## Technical Solution

In accordance with an aspect of the present invention, there is provided a method for imaging collateral circulation in the body, the method including: (a) image post-processing source data of dynamic susceptibility contrast-enhanced perfusion weighted imaging (DSC-PWI) as magnetic resonance imaging in the body; and (b) comparing images obtained through the image post-processing in step (a) with digital subtraction angiography (DSA) images to image collateral circulation.

As used herein, the term "collateral circulation" refers to a situation where the blood flow detours to other blood vessels when a normal blood flow is blocked due to stricture or occlusion of the blood vessel. Cerebral blood flows are composed of anterior circulation by carotid arteries at both sides and posterior circulation by the vertebrobasilar artery at the back side, and theses anastomose in the skull base to form the circle of Willis, and diverge into the anterior cerebral artery, middle cerebral artery, and posterior cerebral artery. These blood vessels are ultimately in charge of the supply of blood to a predetermined region of the brain, but the anastomosis between these blood vessels occurs in some sites to make collateral circulation when a lesion occurs on one blood vessel.

According to a preferable embodiment of the present invention, the collateral circulation of the present invention includes ones at the regions of the brain, heart, leg and arm terminals, and intestine, and more preferably, the collateral circulation of the present invention includes ones occurring at the time of cerebral infarction or myocardial infarction.

As used herein, the term "cerebral infarction" refers to a disease in which the blood vessel of the brain becomes occluded and thus a portion of the brain dies. The cerebral infarction is caused by cerebrovascular occlusion due to blood clots resulting from cerebrovascular arteriosclerosis and emboli derived from the heart.

As used herein, the term "magnetic resonance imaging" refers to a diagnostic technique that provides pictures of the body's internal structures using a magnetic field. The machine that is used for the magnetic resonance imaging forms a very strong magnetic field in the body using magnetic pulses, and images the body's internal structures using signals made during the formation and destruction of such a magnetic field.

As used herein, the term "dynamic susceptibility contrast-enhanced perfusion weighted imaging (DSC-PWI)" refers to one technique of the magnetic resonance imaging that detects the change in the magnetic field, caused by the increase in susceptibility. In general, DSC-PWI is a technique in which gadolinium diethylene triamine penta acetic acid (GD-DTPA) as a paramagnetic contrast agent is intravenously injected into the arm of patient to observe the signal change over time through consecutive photographing, and then images various cerebral blood flow-derived maps, such as cerebral blood flow, cerebral blood flow amount, and mean travel time of the contrast agent, through the detection of the signal change.

As used herein, the term "image post-processing" is a collective term for a series of operations in which, after the acquisition of an image, multiple pieces of information of the image, as source data, are corrected and reconstructed to output a new image. Generally, the medical image may be defined by 3-D function, $f(x, y, z)$, and here, $x$, $y$, and $z$ mean spatial coordinates, and the magnitude of $f$ at the coordinates is called the signal intensity of the image. The medical image is composed of finite components since it is a digital image. Each component has a particular location and value, and is called a voxel. The signal intensity values of the respective voxels are corrected and reconstructed through an image post-processing procedure, and output as an image composed of new pieces of information.

As used herein, the term "digital subtraction angiography (DSA)" refers to one technique of angiography. Specifically, first, a simple X-ray is performed on a site that is to be subjected to angiography, and then a contrast agent, which is observable by an X-ray, is injected to the blood vessel, and then an X-ray is performed thereon. These two kinds of scan information are input to a computer to subtract first scan information from second scan information. Through this procedure, an image in which only the blood vessels are clearly contrasted but the other area is eliminated can be obtained. The technique that obtains an image of the blood vessel more clearly through this manner is called a digital subtraction angiography.

According to the present invention, the image post-processing of the DSC-PWI source data in step (a) comprises step (a)-1 of subtracting a signal of an image obtained before the injection of a contrast agent from signals of images obtained after the injection of the contrast agent to obtain sequential images over time.

As described above, by subtracting the signal of the image obtained before the injection of the contrast agent from the signals of the sequential images over time obtained after the injection of the contrast agent, signals from anatomical structures are eliminated and only signals from the contrast agent are left, and thus the tissue to be imaged can be sequentially visualized. The sequential DSC-PWI images over time mean sequential images having a predetermined time interval, and the predetermined time interval is preferably 0.1-10 seconds, more preferably 0.5-5 seconds, still more preferably 1.0-3 seconds, and most preferably 1.5-2 seconds.

According to the present invention, the image post-processing of DSC-PWI source data in step (a) comprises step (a)-2 of merging adjacent consecutive image cuts in the sequential images obtained in step (a)-1.

Herein, by merging the adjacent consecutive image cuts in the sequential DSC-PWI images, the spatial resolution of the image is improved and the signal intensity change over time is maximized. The number of consecutive image cuts that are merged may be appropriately selected within the range in which the spatial resolution can be improved and the signal intensity change over time can be maximized. For example, the merging of the adjacent consecutive image cuts may be performed by merging two to ten, preferably two to seven, more preferably two to five, still more preferably two to three, and the most preferably two adjacent consecutive image cuts.

According to the present invention, the comparing of the post-processed DSC-PWI images in step (b) and the DSA images is performed on images corresponding to an arterial, capillary, venous, or late venous phase.

As used herein, the term "arterial phase" refers to a phase in which contrast staining starts from the insular region of the cerebral parenchyma of the normal hemisphere; the term "capillary phase" refers to a phase in which the whole cerebral parenchyma of the normal hemisphere is stained at the maximal signal intensity; the term "venous phase" refers to a phase in which the contrast agent is washed out in the cerebral parenchyma of the normal hemisphere; and the term "late venous phase" refers to a phase in which the contrast agent is washed out in the cerebral parenchyma of the normal hemisphere but the remaining contrast is shown in the cerebral parenchyma of the lesional hemisphere.

According to the present invention, the DSC-PWI images in the arterial, capillary, venous, and late venous phases are composed of consecutive "images, each of which is obtained by merging adjacent consecutive image cuts" described as above. More preferably, the DSC-PWI images in the arterial, venous, and late venous phases are composed of two consecutive images, "each of which is obtained by merging adjacent consecutive image cuts" described as above, and the DSC-PWI images in the capillary phase are composed of three consecutive images, "each of which is obtained by merging adjacent consecutive image cuts" described as above.

According the present invention, the DSC-PWI images in the arterial phase are merged to construct an early-phase image of collateral circulation, the DSC-PWI images in the capillary phase are merged to construct a mid-phase image of collateral circulation, and the DSC-PWI images in the venous and late venous phases are merged to construct a late-phase image of collateral circulation.

Features and advantages of the present invention are summarized as follows.

(i) The present invention is directed to a novel magnetic resonance imaging technique for promptly and accurately visualizing collateral circulation in the body.

(ii) In the present invention, the collateral circulation can be visualized by image post-processing source data of dynamic susceptibility contrast-enhanced perfusion weighted imaging (DSC-PWI), and then comparing the images obtained through the post-processing with digital subtraction angiography (DSA) images.

(iii) According to the method of the present invention, the collateral circulation can be non-invasively visualized and evaluated easily and promptly, thereby providing information that is important in understanding, making therapeutic decision, and predicting outcomes, of diseases caused by stricture or occlusion of the blood vessel.

**Advantageous Effects**

The present invention is directed to a novel magnetic resonance imaging technique for visualizing collateral circulation in the body. In the present invention, the collateral circulation is visualized by image post-processing source data of dynamic susceptibility contrast-enhanced perfusion weighted imaging (DSC-PWI), and then comparing the images obtained through the post-processing with digital subtraction angiography (DSA) images. According to the method of the present invention, the collateral circulation can be non-invasively visualized and evaluated easily and promptly, thereby providing information that is important in understanding, making therapeutic decision, and predicting outcomes, of diseases caused by stricture or occlusion of the blood vessel.

**Brief Description of the Drawings**

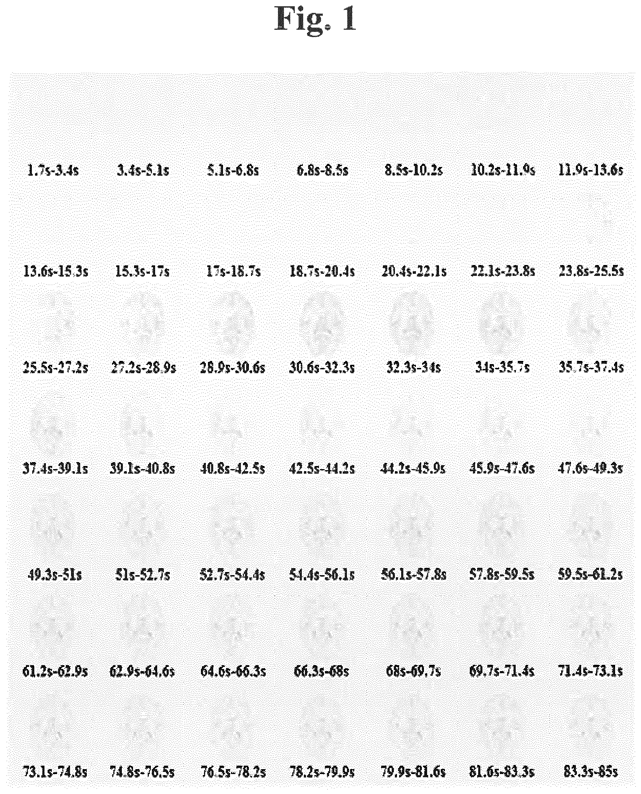

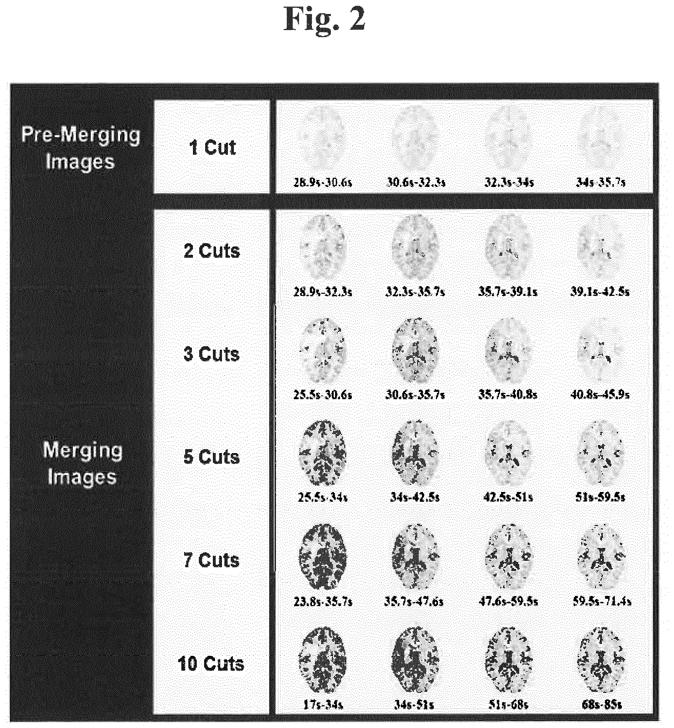

FIG. 1 shows the visualization of brain parenchyma using dynamic susceptibility contrast-enhanced perfusion weighted imaging (DSC-PWI) source data in a patient with right middle cerebral artery occlusion. DSC-PWI source data are composed of 50 image cuts per slice, with a time interval of about 1.7 seconds between image cuts. The time below each image indicates the time elapsed from the start of source data acquisition. It can be seen that contrast staining and washout were relatively slower in the right hemisphere rather than the left hemisphere since the right middle cerebral artery was occluded. FIG. 2 shows the merged results of DSC-PWI image cuts to obtain the best temporal/spatial resolution in the collateral circulation imaging procedure. The best temporal/spatial resolution can be obtained when two adjacent image cuts are merged. Whereas, the spatial resolution is low in the unmerged images, and the temporal resolution is low when more image cuts are merged.

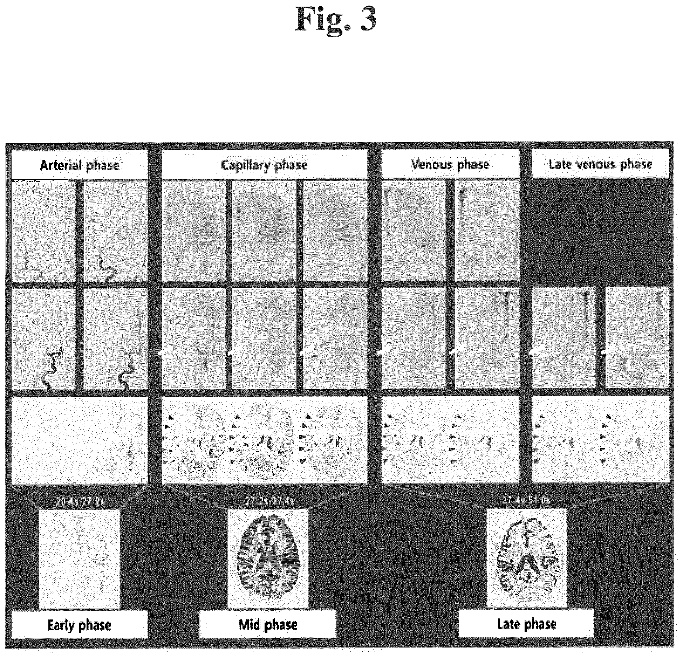

FIG. 3 shows that digital subtraction angiography images are matched to DSC-PWI images to construct collateral circulation images. The first line shows images obtained by digital subtraction angiography performed in the contra-lesional hemisphere, each consisting of arterial, capillary, and venous phases. The second line shows images obtained by digital subtraction angiography performed in the lesional hemisphere, demonstrating proximal right middle cerebral artery occlusion (thin white arrow). In addition, collateral circulation down from the right anterior cerebral artery can be observed from the capillary phase (thick white arrows). Collateral circulation can be observed until the late venous phase. The third line shows images obtained by merging two DSC-PWI image cuts corresponding to each phase of digital subtraction angiography images. Contrast staining and washout by collateral circulation can be confirmed in the lesional hemisphere (black arrowheads). The fourth line shows the generation of collateral circulation images. The images obtained by merging two DSC-PWI image cuts corresponding to the arterial, capillary, venous, and late venous phases are again merged to generate images for the evaluation of collateral circulation. The respective phases correspond to an early phase, a mid-phase, and a late phase.

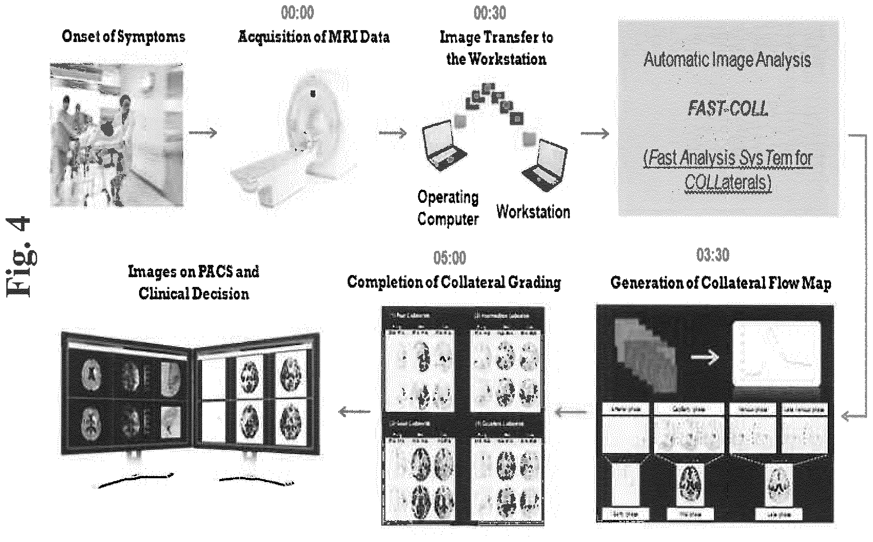

FIG. 4 shows the creation procedure of collateral circulation images using automatic software FAST-COLL. The drawing shows an overall flow from the hospital visit of a patient with the onset of symptoms of cerebral infarction to the generation of collateral circulation images. After brain magnetic resonance imaging is performed, the images are transferred to the workstation, and collateral circulation images are generated using FAST-COLL, and, based on the results, the collateral status is evaluated. The generated collateral circulation images are transferred to the picture archiving communication system (PACS), and thus, various pieces of information with respect to the patient, together with pieces of information from the multi-modal magnetic resonance imaging are provided. When FAST-COLL is used, it takes about 5 minutes from the acquisition of magnetic resonance imaging source data to the evaluation thereof.

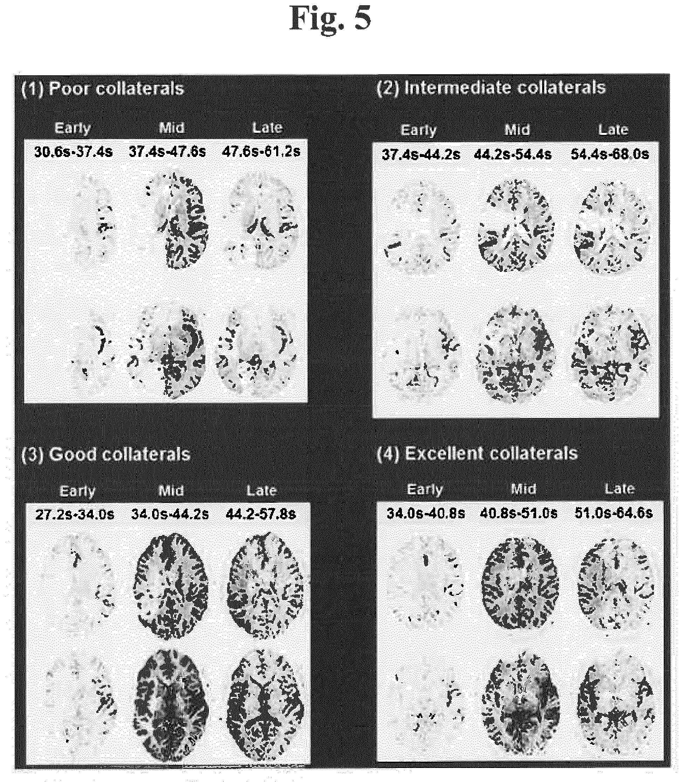

FIG. 5 shows evaluation results of collateral status in collateral circulation images. Panel (1) shows images evaluated as poor collaterals, indicating that contrast staining in the lesional hemisphere is completely non-visible until the late phase or occurs with slow collateral circulation in only some regions. Panel (2) shows images evaluated as intermediate collaterals, indicating that contrast staining in the lesional hemisphere is completely non-visible in some regions and occurs with fast collateral circulation in other some regions. Panel (3) shows images evaluated as good collaterals, indicating that contrast staining in the lesional hemisphere completely occurs with slow collateral circulation. Panel (4) shows images evaluated as excellent collaterals, indicating that contrast staining completely occurs with fast collateral circulation.

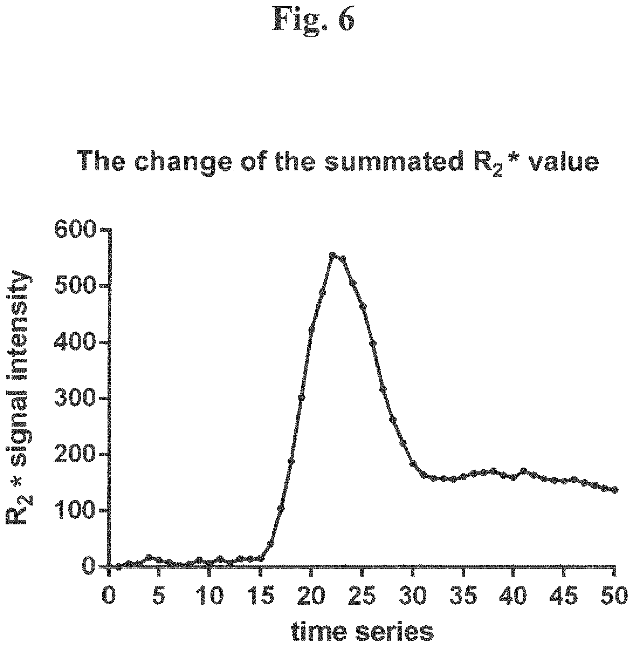

FIG. 6 is a graph showing the change in the summated $R_2{}^*$ value over time, the summated $R_2{}^*$ value being obtained by adding $R_2{}^*$ values of all voxels in each cut of DSC-PWI source data. The $R_2{}^*$ value is calculated by equation $R_2{}^*(t) = -1/TE \log [S(t)/S_o]$, wherein $S(t)$ is the magnetic resonance signal intensity at the corresponding time, $S_o$ is the baseline magnetic resonance signal intensity, and TE is the echo time.

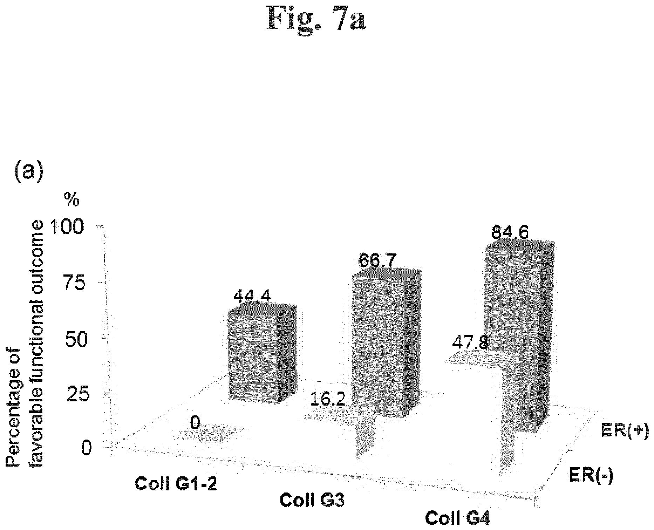

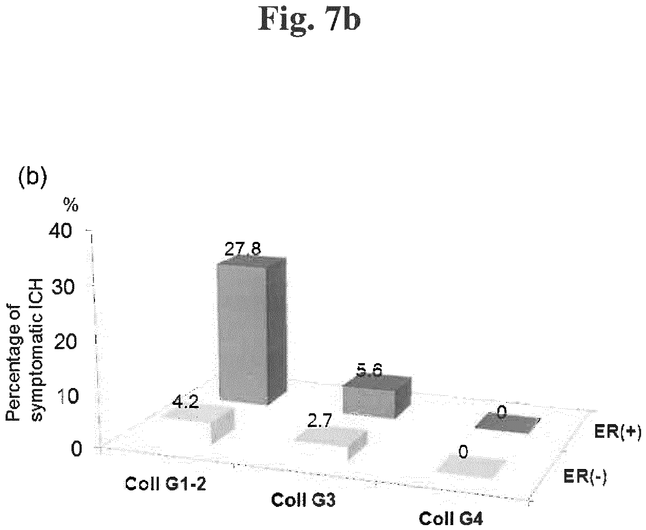

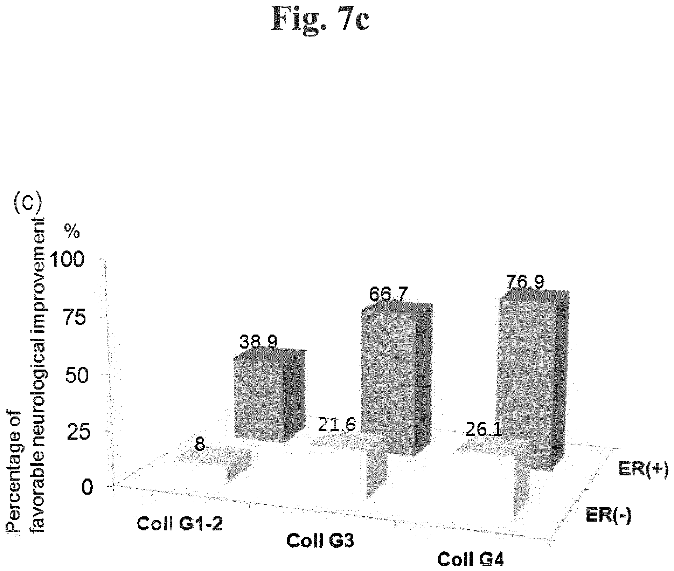

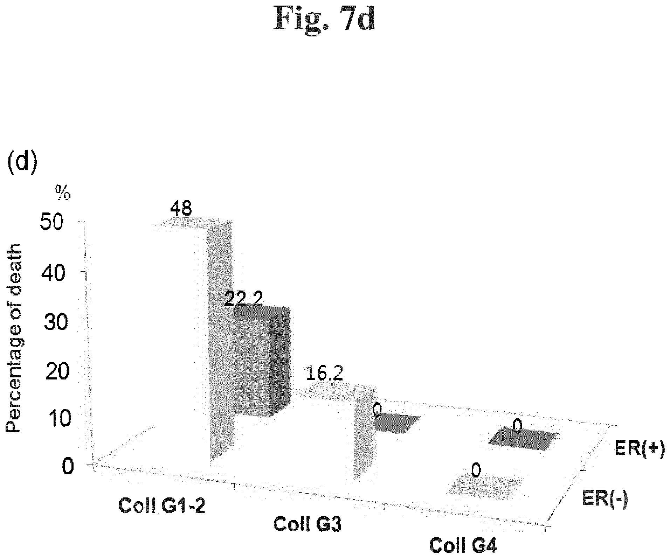

FIGS. 7a to 7d are graphs showing collateral grades observed in collateral circulation images and outcomes after cerebral infarction according to blood vessel recanalization. ER (early recanalization) indicates blood vessel recanalization, Coll (collaterals) indicates collateral circulation, and G (grade) indicates grade. Collateral grades G1, G2, G3, and G4 correspond to poor collaterals, intermediate collaterals, good collaterals, and excellent collaterals, respectively, (see example 5 and figure 5). As shown in FIG. 7a, the higher collateral grade is more likely to have a favorable functional outcome. As shown in FIG. 7b, the higher collateral grade is more likely to have a low occurrence of symptomatic cerebral hemorrhage. As shown in FIG. 7c, the higher collateral grade is more likely to have a favorable neurological improvement. As shown in FIG. 7d, the higher collateral grade is more likely to have a low death rate.

## Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

## EXAMPLES

### Example 1: Sequential visualization of brain parenchyma by gadolinium contrast agent in DSC-PWI

As a first step for visualizing collateral circulation, contrast staining and washout in the brain parenchyma was sequentially visualized using DSC-PWI source data (FIG. 1). To this end, among 50 images obtained for each slice of DSC-PWI source data, the signal of the first image cut was subtracted from the signals of the other 49 image cuts, respectively. Thus, the signals from anatomical structures were eliminated and only the effect by the gadolinium contrast agent was left, thereby allowing sequential visualization of the brain parenchyma.

### Example 2: Image merging for obtaining best temporal and spatial resolution

As a second step for imaging collateral circulation, the images obtained from DSC-PWI were sequentially merged stage by stage, and then a comparision of temporal and spatial resolution was performed (FIG. 2). To this end, the images obtained through example 1 were merged across two, three, five, or ten consecutive cuts. Although raw images before being merged had the best temporal resolution, differences between both hemispheres on a single cut, as well as between adjacent cuts, were less prominent than those in the merged images. Meanwhile, the images obtained by merging five or more cuts provided excellent spatial resolution, but the change in signal intensity over time could not be precisely determined. The images obtained by merging two cuts provided better temporal resolution than the images obtained by merging three cuts while providing similar spatial resolution therebetween. Therefore, the images obtained by merging two adjacent cuts were finally used for collateral circulation imaging.

### Example 3: Generation of collateral circulation images

As the last step for visualizing collateral circulation, the two-cut merged images determined in example 2 were matched to images obtained by digital subtraction angiography (FIG. 3). To this end, the intensity of contrast staining and washout of the brain parenchyma in the normal and the lesional hemispheres were visually determined on the two-cut merged images to select images corresponding to the arterial, capillary, and venous phases in digital subtraction angiography. Specifically, the arterial phase started showing contrast staining from the insular region of the brain parenchyma of the normal hemisphere; the capillary phase demonstrated the whole brain parenchyma staining with maximal signal intensity in the normal hemisphere; the venous phase revealed the washout of the contrast agent in the brain parenchyma of the normal hemisphere; and the late venous phase demonstrated the contrast agent remaining in the brain parenchyma of the lesional hemisphere while the contrast agent was washed out in the brain parenchyma of the normal hemisphere. Typically, the capillary phase was composed of three images of two-merged consecutive cuts, and the other phases, that is, the arterial, venous, and late venous phases, were composed of two images of two-merged consecutive cuts. The collateral circulation images were generated by again merging two images of two-merged cuts in the arterial phase, three images of two-merged cuts in the capillary phase, and four images of two-merged cuts in the venous and late venous phases, respectively. The phases of the thus generated collateral circulation images correspond to early, mid, and late phases, respectively.

## Example 4: Creation of collateral circulation images using automatic software

Collateral circulation images were automatically generated using the same logic algorithm as in the method of obtaining collateral circulation images described in example 3. This automatic software was named a fast analysis system for collaterals (FAST-COLL). The overall flow for generating collateral circulation images using FAST-COLL is shown in FIG. 4. FAST-COLL calculates the $R_2^*$ value at each voxel in DSC-PWI source data, and the following formula was used:

$$R_2^*(t) = -\frac{1}{TE}\log\left(\frac{S(t)}{S_0}\right)$$

where S(t) is the magnetic resonance signal intensity at the corresponding time; $S_0$ is the baseline magnetic resonance signal intensity; and TE is the echo time. For the creation of collateral circulation images, all $R_2^*$ values at every voxel on each cut are summed, and the change in the summated $R_2^*$ value over time was plotted on a graph (FIG. 6). The maximum summated $R_2^*$ value was automatically found from the graph, and the cut corresponding to the maximum value was set as the midpoint of the mid-phase of collateral circulation images. Using this cut as a reference, collateral circulation images were generated by merging the adjacent images, as in example 3.

## Example 5: Evaluation of collateral status on collateral circulation images

After the collateral circulation images were generated, the collateral status was determined from the generated images. The collateral status may be largely classified into no collateral circulation, slow collateral circulation, and fast collateral circulation. No collateral circulation indicates that contrast staining is completely non-visible until the late phase; slow collateral circulation indicates that contrast staining is non-visible in the mid-phase but is visible in the late phase; and fast collateral circulation indicates that contrast staining is visible in the mid-phase in the lesional hemisphere, similarly to the normal hemisphere. Based on this matter, the collateral circulation may be evaluated. As shown in collateral circulation images in the FIG. 5, it may be evaluated as poor collaterals if contrast staining in the lesional hemisphere is completely non-visible until the late phase or occurs with slow collateral circulation only in some regions; it may be evaluated as intermediate collaterals if contrast staining in the lesional hemisphere is completely non-visible in some regions and occurs with fast collateral circulation in other some regions; it may be evaluated as good collaterals if contrast staining in the lesional hemisphere completely occurs with slow collateral circulation; and it may be evaluated as excellent collaterals if contrast staining completely occurs with fast collateral circulation.

## Example 6: Predicting outcomes according to collateral grade obtained from collateral circulation images in patients with acute cerebral infarction after blood vessel recanalization therapy

The outcomes deepening on the recanalization or not of blood vessels can be predicted according to the collateral grade obtained from collateral circulation images in patients with acute cerebral infarction after blood vessel recanalization therapy. As shown in FIGS. 7a to 7d, regardless of the achievement of recanalization of blood vessels in acute cerebral infarction, the higher collateral grade is more likely to have a favorable outcome, a low occurrence of symptomatic cerebral hemorrhage, and a low death rate. That is, when the collateral grade observed on the collateral circulation images is high, the recanalization of blood vessels can lead to a prediction of a favorable outcome without a risk of symptomatic cerebral hemorrhage.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims.

**Claims**

1. A computer implemented method for imaging collateral circulation in the body, the method comprising:

   (a) image post-processing source data of dynamic susceptibility contrast-enhanced perfusion weighted imaging (DSC-PWI) as magnetic resonance imaging in the body comprising subtracting a signal of an image obtained before the injection of a contrast agent from signals of images obtained after the injection of the contrast agent to obtain sequential images over time;
   (b) merging two to ten adjacent consecutive image cuts in the sequential images obtained in step (a);
   (c) comparing images obtained through the image post-processing in step (a) with digital subtraction angiography (DSA) images to image collateral circulation,

   wherein the comparing of the DSC-PWI images and the DSA image is performed on images corresponding to the arterial, capillary, venous, or late venous phase, wherein the DSC-PWI images in the arterial, capillary, venous, and late venous phases are composed of consecutive images, each of which is obtained by merging adjacent consecutive image cuts, and
   wherein the DSC-PWI images of the arterial phase are merged to construct a collateral circulation image in the early phase; the DSC-PWI images of the capillary phase are merged to construct a collateral circulation image in the mid phase; and the DSC-PWI images of the venous and late venous phases are merged to construct a collateral circulation image in the late phase.

2. The method of claim 1, wherein the collateral circulation is collateral circulation in the brain or heart region.

**Patentansprüche**

1. Computerimplementiertes Verfahren zur Bildgebung des Kollateralkreislaufs im Körper, umfassend:

   (a) Bildnachbearbeiten von Quelldaten der dynamischen suszeptibilitätskontrastverstärkten perfusionsgewichteten Bildgebung (engl. *dynamic susceptibility contrast-enhanced perfusion weighted imaging,* DSC-PWI) als Magnetresonanzbildgebung im Körper, umfassend das Subtrahieren eines Signals eines vor der Injektion des Kontrastmittels erhaltenen Bildes von Signalen von nach der Injektion des Kontrastmittels erhaltenen Bildern, um sequentielle Bilder über die Zeit zu erhalten;
   (b) Zusammenführen von zwei bis zehn benachbarten konsekutiven Bildschnitten in den in Schritt (a) erhaltenen sequenziellen Bildern;
   (c) Vergleichen von durch die Bildnachbearbeitung in Schritt (a) erhaltenen Bildern mit digitalen Subtraktionsangiographie (DSA)-Bildern, um den Kollateralkreislauf abzubilden,

wobei das Vergleichen der DSC-PWI-Bilder und des DSA-Bildes an Bildern durchgeführt wird, die der arteriellen, kapillaren, venösen oder spätvenösen Phase entsprechen,
wobei die DSC-PWI-Bilder in den arteriellen, kapillaren, venösen und spätvenösen Phasen aus konsekutiven Bildern bestehen, von denen ein jedes erhalten wurde, indem benachbarte konsekutive Bildschnitte zusammengeführt wurden, und
wobei die DSC-PWI-Bilder der arteriellen Phase zusammengeführt werden, um ein Kollateralkreislaufbild in der Frühphase zu konstruieren; die DSC-PWI-Bilder der kapillaren Phase zusammengeführt werden, um ein Kollateralkreislaufbild in der mittleren Phase zu konstruieren; und die DSC-PWI-Bilder der venösen und spätvenösen Phase zusammengeführt werden, um ein Kollateralkreislaufbild in der Spätphase zu konstruieren.

2. Verfahren nach Anspruch 1, wobei der Kollateralkreislauf der Kollateralkreislauf im Gehirn oder in der Herzregion ist.

**Revendications**

1. Procédé mis en œuvre par ordinateur pour l'imagerie de circulation collatérale dans le corps, ledit procédé comprenant :

   (a) des données sources de post-traitement d'image de l'imagerie pondérée de perfusion par amélioration de contraste de susceptibilité dynamique (DSC-PWI) sous forme d'imagerie par résonance magnétique dans le corps comprenant la soustraction d'un signal d'une image obtenue avant l'injection d'un agent de contraste provenant de signaux d'images obtenues après l'injection de l'agent de contraste pour obtenir des images séquentielles avec le temps;
   (b) la fusion de deux à dix coupes d'images consécutives adjacentes dans les images séquentielles obtenues à l'étape (a);
   (c) la comparaison des images obtenues par post-traitement des images à l'étape (a) avec les images d'angiographie par soustraction numérique (DSA) à la circulation collatérale des images,

la comparaison des images DSC-PWI et de l'image DSA étant effectuée sur des images correspondant à la phase artérielle, capillaire, veineuse ou veineuse tardive,

les images DSC-PWI dans la phase artérielle, capillaire, veineuse et veineuse tardive étant composées d'images consécutives, chacune étant obtenue par fusion de coupes d'images consécutives adjacentes, et

les images DSC-PWI de la phase artérielle étant fusionnées pour construire une image de circulation collatérale dans la phase initiale; les images DSC-PWI de la phase capillaire étant fusionnées pour construire une image de circulation collatérale dans la phase médiane; et les images DSC-PWI des phases veineuse et veineuse tardive étant fusionnées pour construire une image de circulation collatérale dans la phase tardive.

2. Procédé de la revendication 1, la circulation collatérale étant la circulation collatérale dans le cerveau ou la région cardiaque.

# Fig. 1

| 1.7s-3.4s | 3.4s-5.1s | 5.1s-6.8s | 6.8s-8.5s | 8.5s-10.2s | 10.2s-11.9s | 11.9s-13.6s |

| 13.6s-15.3s | 15.3s-17s | 17s-18.7s | 18.7s-20.4s | 20.4s-22.1s | 22.1s-23.8s | 23.8s-25.5s |

| 25.5s-27.2s | 27.2s-28.9s | 28.9s-30.6s | 30.6s-32.3s | 32.3s-34s | 34s-35.7s | 35.7s-37.4s |

| 37.4s-39.1s | 39.1s-40.8s | 40.8s-42.5s | 42.5s-44.2s | 44.2s-45.9s | 45.9s-47.6s | 47.6s-49.3s |

| 49.3s-51s | 51s-52.7s | 52.7s-54.4s | 54.4s-56.1s | 56.1s-57.8s | 57.8s-59.5s | 59.5s-61.2s |

| 61.2s-62.9s | 62.9s-64.6s | 64.6s-66.3s | 66.3s-68s | 68s-69.7s | 69.7s-71.4s | 71.4s-73.1s |

| 73.1s-74.8s | 74.8s-76.5s | 76.5s-78.2s | 78.2s-79.9s | 79.9s-81.6s | 81.6s-83.3s | 83.3s-85s |

# Fig. 2

| | | | | | |
|---|---|---|---|---|---|
| **Pre-Merging Images** | **1 Cut** | 28.9s-30.6s | 30.6s-32.3s | 32.3s-34s | 34s-35.7s |
| **Merging Images** | **2 Cuts** | 28.9s-32.3s | 32.3s-35.7s | 35.7s-39.1s | 39.1s-42.5s |
| | **3 Cuts** | 25.5s-30.6s | 30.6s-35.7s | 35.7s-40.8s | 40.8s-45.9s |
| | **5 Cuts** | 25.5s-34s | 34s-42.5s | 42.5s-51s | 51s-59.5s |
| | **7 Cuts** | 23.8s-35.7s | 35.7s-47.6s | 47.6s-59.5s | 59.5s-71.4s |
| | **10 Cuts** | 17s-34s | 34s-51s | 51s-68s | 68s-85s |

# Fig. 3

# Fig. 4

Onset of Symptoms

Acquisition of MRI Data — 00:00

Image Transfer to the Workstation — 00:30

Operating Computer    Workstation

Automatic Image Analysis
*FAST-COLL*
(*Fast Analysis SysTem for COLLaterals*)

Generation of Collateral Flow Map — 03:30

Completion of Collateral Grading — 05:00

Images on PACS and Clinical Decision

## Fig. 5

# Fig. 6

The change of the summated $R_2$* value

# Fig. 7a

(a)

# Fig. 7b

(b)

# Fig. 7c

# Fig. 7d

**EP 3 005 943 B1**

**REFERENCES CITED IN THE DESCRIPTION**

**Non-patent literature cited in the description**

- **BANG OY et al.** *J Neurol Neurosurg Psychiatry,* 2008, vol. 79, 625
- **BANG OY et al.** *Stroke,* 2011, vol. 42, 693
- **BANG OY et al.** *Stroke,* 2011, vol. 42, 2235